# EUROPÄISCHE PATENTSCHRIFT

(11) **EP 2 825 222 B1**
(45) Veröffentlichungstag und Bekanntmachung des Hinweises auf die Patenterteilung: **20.12.2017**
(21) Anmeldenummer: 13711289.2
(22) Anmeldetag: 13.03.2013
(51) Int. Cl.: A61M 1/34

(54) **BLUTBEHANDLUNGSVORRICHTUNG ZUM DOSIEREN EINES MITTELS DIESER ERZEUGTEN SUBSTITUATS**
BLOOD TREATMENT DEVICE FOR DOSING A SUBSTITUATE GENERATED BY THE DEVICE
DISPOSITIF DE TRAITEMENT DE SANG POUR DOSAGE D'UN SUBSTITUT GÉNÉRÉ AU MOYEN DU DISPOSITIF

(30) Priorität: 14.03.2012 DE 102012004970; 14.03.2012 US 201261610485 P
(43) Veröffentlichungstag der Anmeldung: 21.01.2015
(73) Patentinhaber: Fresenius Medical Care Deutschland GmbH, 61352 Bad Homburg (DE)
(72) Erfinder: GRONAU, Soeren, 65428 Rüsselsheim (DE); HAECKER, Juergen, 61267 Neu-Anspach (DE); MUELLER, Ralf, 61350 Bad Homburg (DE)
(74) Vertreter: Bobbert & Partner Patentanwälte PartmbB
(86) Internationale Anmeldenummer: PCT/EP2013/000747
(87) Internationale Veröffentlichungsnummer: WO 2013/135379

(56) Entgegenhaltungen:
- EP-A1- 0 763 367
- EP-A1- 0 930 080
- EP-A2- 0 694 312
- DE-A1- 19 700 466
- DE-A1-102010 032 179
- US-A1- 2003 073 945

## Beschreibung

Die vorliegende Erfindung betrifft eine Blutbehandlungsvorrichtung zum Dosieren, Erzeugen oder Bereitstellen eines mittels dieser erzeugten Substituats gemäß Anspruch 1.

Aus der Praxis sind extrakorporale Blutbehandlungsvorrichtungen und -verfahren bekannt, bei welchen dem extrakorporalen Blutkreislauf ein Substituat zugegeben wird. Dieses Substituat wird regelmäßig online, d. h. während der Blutbehandlungssitzung und in aller Regel mittels der Blutbehandlungsvorrichtung selbst, hergestellt. Hierzu wird ein Teil der in aller Regel ebenfalls online hergestellten Dialysierflüssigkeit, nachdem diese durch die Membran eines ersten Filters oder einer ersten Filterstufe durchgetreten ist, veranlasst, auch durch die Membran eines zweiten Filters oder einer zweiten Filterstufe durchzutreten. Nach Durchlaufen dieser zweiten Filtration (Sterilfiltration) kann das auf diese Weise gewonnene Substituat an einer Prädilutionsstelle und/oder an einer Postdilutionsstelle des extrakorporalen Blutkreislaufs stromaufwärts bzw. stromabwärts des Dialysators oder Blutfilters zum Inhalt des extrakorporalen Blutkreislaufs hinzugefügt werden.

An die Genauigkeit, mit welcher das Substituat dem Inhalt des extrakorporalen Blutkreislaufs hinzudosiert wird, werden Anforderungen gestellt. Daher weisen Vorrichtungen des Standes der Technik, wie sie aus der Praxis bekannt sind, eine hochgenau arbeitende Dosiervorrichtung zum Dosieren des Substituats auf, welche ganz (z. B. in Form einer Membranpumpe in einer Blutkassette) oder teilweise (z. B. in Form eines Pumpschlauchsegments für eine Rollenpumpe an einem herkömmlichen Blutschlauchsatz oder integriert in eine Blutkassette) Teil des zur Blutbehandlung verwendeten Blutschlauchsatzes sind.

So ist beispielsweise aus der DE 197 00 466 A1 eine Vorrichtung zur Hämodiafiltration bekannt, welche eine Flüssigkeitsbilanziereinrichtung aufweist und online Substituatlösung aus Dialysat mithilfe von Sterilfiltern aufbereitet, wobei die Vorrichtung zur Hämodiafiltration einen ersten und einen zweiten Filtern aufweist und der zweite Filter zwischen dem ersten Filter und dem Dialysator in einem Überdruckbereich angeordnet ist.

Aus der EP 0 930 080 A1 sind ein Verfahren sowie eine Vorrichtung zur Bereitstellung betriebsbereiter Dialysierflüssigkeit bekannt.

In der EP 0 763 367 A1 wird ein Verfahren zum Überprüfen auf Undichtigkeiten in einem Dialysierflüssigkeitssystem beschrieben.

In der US 2033/0073945 A1 wird eine Hämodiafiltrationsvorrichtung beschrieben.

Die DE 10 2010 032 179 A1 offenbart einen Konnektor zum Verbinden von zwei medizinischen Systemen.

Eine Aufgabe der vorliegenden Erfindung ist es, eine entsprechende Blutbehandlungsvorrichtung vorzuschlagen.

Die erfindungsgemäße Aufgabe wird durch eine Blutbehandlungsvorrichtung mit den Merkmalen des Anspruchs 1 gelöst.

Erfindungsgemäß wird somit eine Blutbehandlungsvorrichtung zum Ausführen eines Verfahren zum Dosieren, Erzeugen oder Bereitstellen eines mittels einer Blutbehandlungsvorrichtung erzeugten Substituats vorgeschlagen. Das Verfahren erfolgt dabei mittels einer Hydraulik oder eines Hydraulikabschnitts (im Folgenden kurz: Hydraulik) der Blutbehandlungsvorrichtung. Die Hydraulik weist wenigstens eine in die Dialysatkammer eines Blutfilters oder eines Dialysators (im Folgenden kurz: Dialysator) mündende oder dem Dialysator Dialysierflüssigkeit zuführende Dialysierflüssigkeitszulaufleitung auf. Die Hydraulik weist ferner wenigstens eine Substituatleitung sowie eine erste Filterstufe und eine zweite Filterstufe auf.

Die Dialysierflüssigkeitszulaufleitung führt dem Dialysator unverbrauchte Dialysierflüssigkeit zu, wohingegen die weiter unten erwähnte Dialysatablaufleitung verbrauchte Dialysierflüssigkeit, auch als Dialysat bezeichnet, aus dem Dialysator abführt.

Die Substituatleitung führt Substituat, welches dem Inneren oder dem Inhalt eines bei der Blutbehandlung verwendeten Blutkreislaufs (im Wesentlichen Blut) zur Volumensubstitution zugeführt wird, oder sie ist hierzu vorgesehen.

Optional kann die Hydraulik ferner eine Abzweigleitung aufweisen, welche die Dialysierflüssigkeitszulaufleitung mit der Substituatleitung verbindet. Dies erfolgt optional unter Einschaltung der zwischen der Abzweigleitung und der Substituatleitung liegenden, im Folgenden als zweite Filterstufe bezeichneten Filterstufe. Die Abzweigleitung mündet in die zweite Filterstufe, ist mit dieser verbunden und/oder führt unverbrauchte Dialysierflüssigkeit in diese hinein. Der hierin anstelle von "Filterstufe" auch verwendete Begriff "Filter" kann als Synonym zur Filterstufe verstanden werden. Eine Filterstufe kann wiederum aus mehreren Filtern bestehen.

Das Verfahren umfasst ein Fördern eines ersten Fluids durch die erste Filterstufe, welche der zweiten Filterstufe in Strömungsrichtung vorgelagert ist, hindurch (oder ein Filtern des ersten Fluids) und in die Dialysierflüssigkeitszulaufleitung hinein. Dabei verlässt das Fluid die erste Filterstufe als eine in einen Dialysator einbringbare und/oder eingebrachte Dialysierflüssigkeit.

Das Verfahren umfasst ferner ein (aktives oder passives) Leiten eines Anteils (eines Flussanteils oder eines Volumenanteils) der Dialysierflüssigkeit in die Substituatleitung hinein, welche sich an eine zweite Filterstufe anschließt oder stromabwärts hiermit in Fluidkommunikation steht, beispielsweise indem sich die Substituatleitung an die zweite Filterstufe anschließt oder von dieser gespeist wird.

Beim Durchtritt durch die zweite Filterstufe wird ein in einen extrakorporalen Blutkreislauf einbringbares und/oder eingebrachtes Substituat erzeugt.

Das Verfahren umfasst ferner ein Regeln oder Steuern des Anteils der die erste Filterstufe verlassenden Dialysierflüssigkeit (oder der Größe dieses Anteils), welcher in der zweiten Filterstufe gefiltert wird oder deren Membran durchtritt, oder des Anteils, welcher nach Filterung an der zweiten Filterstufe über die Substituatleitung in eine medizinische Funktionsvorrichtung geführt wird oder vorgesehen ist, in das extrakorporal geführte Blut eines Patienten geführt zu werden.

Das Regeln oder Steuern des Anteils der Dialysierflüssigkeit oder des Substituats, der jeweils die zweite Filterstufe durchtritt oder in die Funktionsvorrichtung (oder in einen extrakorporalen Blutkreislauf) geführt wird, geschieht, indem auf wenigstens eine Fördervorrichtung und/oder wenigstens eine Flussbegrenzungseinrichtung und/oder ein Flussteilerventil eingewirkt wird, welche jeweils in der Dialysierflüssigkeitszulaufleitung, der Substituatleitung und/oder der Abzweigleitung vorliegen oder auf diese wirken.

Das Einwirken kann durch bekannte Maßnahmen zum Betätigen von Flussbegrenzungsvorrichtungen oder Fördereinrichtungen erfolgen.

Die Steuereinrichtung, welche auch als Regeleinrichtung ausgestaltet sein kann, ist vorgesehen, eingerichtet, programmiert und/oder konfiguriert zum Steuern oder Regeln einer Blutbehandlungsvorrichtung zum oder beim Durchführen des Verfahrens bei Zusammenwirken mit der Blutbehandlungsvorrichtung. Hierzu ist sie bei ihrem Gebrauch mit den zu steuernden oder zu regelnden Bauteilen der Hydraulik der Blutbehandlungsvorrichtung verbunden oder steht mit diesen in Wirk- und/oder Signalverbindung.

Die erfindungsgemäße Blutbehandlungsvorrichtung weist eine Hydraulik auf, welche wenigstens eine Dialysierflüssigkeitszulaufleitung und wenigstens eine Substituatleitung aufweist. Die Hydraulik kann optional wenigstens eine wie oben beschriebene Abzweigleitung aufweisen. Die Blutbehandlungsvorrichtung ist ausgestaltet, vorgesehen und/oder konfiguriert zum Durchführen des Dosierverfahrens. Hierzu erforderliche Einrichtungen weist sie in bestimmten Ausführungsformen auf oder ist mit solchen jeweils in Wirk- und/oder Signalverbindung verbunden.

Die medizinische (d. h. für medizinische Zwecke vorgesehene) Funktionsvorrichtung ist zur gemeinsamen Verwendung mit einer erfindungsgemäßen Blutbehandlungsvorrichtung vorgesehen. Sie weist eine eigene Substituatleitung und einen Substituatport oder -anschluss auf, wobei der Substituatport vorgesehen ist, um von der Hydraulik der Blutbehandlungsvorrichtung erzeugtes Substituat aus der Substituatleitung der Hydraulik zu empfangen. Die medizinische Funktionsvorrichtung weist keine Vorrichtung auf, welche angeordnet und/oder vorgesehen ist für eine Dosierung des von der Substituatleitung der Funktionsvorrichtung in eine Blut führende Leitung der Funktionsvorrichtung übertretenden Substituats.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

In manchen erfindungsgemäßen Ausführungsformen ist das Dosieren des Substituats ein maschinelles Erzeugen, Freigeben, Bewirken des Abtrennens oder Zurverfügungstellen eines konkreten Substituatflusses (etwa in Milliliter pro Minute) oder eines konkreten Volumens an Substituatflüssigkeit. Der auf diese Weise mittels der Hydraulik festgelegte Substituatfluss oder das auf diese Weise festgelegte Substituatvolumen entspricht in bestimmten erfindungsgemäßen Ausführungsformen jenem Fluss oder Volumen, welcher bzw. welches die Hydraulik verlassend in eine mit der Blutbehandlung zum Zweck der Blutbehandlungssitzung verbundene medizinische Funktionsvorrichtung eintritt, beispielsweise über einen Substituatport der Hydraulik.

In bestimmten erfindungsgemäßen Ausführungsformen ist die Fördereinrichtung eine Pumpe, eine Druckpumpe, eine Flusspumpe oder eine Volumenpumpe oder dergleichen.

In manchen erfindungsgemäßen Ausführungsformen ist die Flussbegrenzungseinrichtung eine Drossel, ein Flussteilerventil, ein Proportionalventil, ein Schlauchquetschventil oder dergleichen.

In einigen Ausführungsformen des Verfahrens oder der erfindungsgemäßen Blutbehandlungsvorrichtung sind alle zum Dosieren des tatsächlich in den extrakorporalen Blutkreislauf eingebrachten Substituats ausschließlich Bestandteil der Hydraulik oder hierin eingebettet. Dies gilt insbesondere für die im Zusammenhang mit den hierin beschriebenen erfindungsgemäßen Ausführungsformen genannten Fördereinrichtungen wie eine Pumpe, eine Druckpumpe, eine Flusspumpe oder eine Volumenpumpe oder dergleichen. Dies gilt insbesondere auch für die im Zusammenhang mit den hierin beschriebenen erfindungsgemäßen Ausführungsformen genannten Flussbegrenzungseinrichtungen wie eine Drossel, ein Flussteilerventil, ein Proportionalventil, ein Schlauchquetschventil oder dergleichen.

In bestimmten erfindungsgemäßen Ausführungsformen ist die zweite Filterstufe in die Dialysierflüssigkeitszulaufleitung eingefügt, sodass die gesamte Menge an Dialysierflüssigkeit, welche dem Dialysator zugeführt wird, zuvor auch durch die zweite Filterstufe geströmt ist, gleich ob sie hierin gefiltert wurde oder nicht.

In manchen erfindungsgemäßen Ausführungsformen weist die Hydraulik eine zweite Filterstufe in der Abzweigleitung der Dialysierflüssigkeitszulaufleitung oder als Endpunkt der Abzweigleitung auf oder mündet in diese hinein.

In bestimmten erfindungsgemäßen Ausführungsformen weist die Hydraulik wenigstens eine Flushleitung auf. Unter einer "Flushleitung" wird erfindungsgemäß eine Leitung verstanden, welche am Ausgang der Dialysierflüssigkeitskammer der zweiten Filterstufe ansetzt. Diese kann exemplarisch mit der Dialysatablaufleitung des Dialysators verbunden sein. Eine Flushleitung kann erfindungsgemäß auch als Spülleitung, Durchspülleitung oder Freispülleitung bezeichnet oder verwendet werden.

In manchen erfindungsgemäßen Ausführungsformen weist die Hydraulik wenigstens eine Bypassleitung auf, abgehend von der Substituatleitung zum Flush. Diese kann exemplarisch mit der Dialysatablaufleitung des Dialysators verbunden sein.

In einigen erfindungsgemäßen Ausführungsformen wird unter "Flush" ein nur kurzzeitiges Spülen, Freispülen oder Durchspülen verstanden. Dieses kann zum Entfernen von Luft oder Partikeln aus dem Filter gedacht sein. Ein hierzu geöffnetes Ventil kann anschließend geschlossen werden, und die Substitution kann fortgesetzt werden.

In bestimmten erfindungsgemäßen Ausführungsformen weist die Hydraulik ein Flussteilerventil in der Dialysierflüssigkeitszulaufleitung stromabwärts oder "nach" der ersten Filterstufe auf.

In manchen erfindungsgemäßen Ausführungsformen weist die Hydraulik ein Proportionalventil in der Dialysierflüssigkeitszulaufleitung stromabwärts oder nach der zweiten Filterstufe auf.

In bestimmten erfindungsgemäßen Ausführungsformen weist die Hydraulik ein Proportionalventil in der Substituatleitung stromabwärts der zweiten Filterstufe auf.

In gewissen erfindungsgemäßen Ausführungsformen weist die Hydraulik (wenigstens) ein Proportionalventil vor der zweiten Filterstufe und/oder nach der zweiten Filterstufe auf.

In manchen erfindungsgemäßen Ausführungsformen weist die Hydraulik wenigstens eine Vorrichtung zum Messen des Flusses (Flussmessung) in der Dialysierflüssigkeitszulaufleitung auf.

In bestimmten erfindungsgemäßen Ausführungsformen weist die Hydraulik wenigstens eine Vorrichtung zum Messen des Flusses in der Abzweigleitung und stromaufwärts oder "vor" der zweiten Filterstufe auf.

In manchen erfindungsgemäßen Ausführungsformen weist die Hydraulik wenigstens eine Vorrichtung zum Messen des Flusses in der Substituatleitung und stromabwärts der zweiten Filterstufe auf.

In bestimmten erfindungsgemäßen Ausführungsformen weist die Hydraulik wenigstens eine Vordruckpumpe in der Dialysierflüssigkeitszulaufleitung und nach der ersten Filterstufe, aber stromaufwärts eines Abzweigungspunkt, an welchem eine Abzweigleitung von der Dialysierflüssigkeitszulaufleitung abgeht und/oder stromaufwärts der zweiten Filterstufe auf.

In manchen erfindungsgemäßen Ausführungsformen weist die Hydraulik wenigstens eine Vorrichtung zum Messen des Vordrucks (Vordruckmessung) in der Dialysierflüssigkeitszulaufleitung und stromaufwärts der zweiten Filterstufe auf.

In bestimmten erfindungsgemäßen Ausführungsformen weist die Hydraulik wenigstens eine Vordruckpumpe in der Abzweigleitung und stromaufwärts der zweiten Filterstufe auf.

In manchen erfindungsgemäßen Ausführungsformen weist die Hydraulik wenigstens eine Druckpumpe in der Substituatleitung und stromabwärts der zweiten Filterstufe auf.

In bestimmten erfindungsgemäßen Ausführungsformen weist die Hydraulik wenigstens eine Volumenpumpe in der Abzweigleitung und stromaufwärts der zweiten Filterstufe auf.

In manchen erfindungsgemäßen Ausführungsformen weist die Hydraulik wenigstens eine Volumenpumpe in der Abzweigleitung und stromaufwärts der zweiten Filterstufe auf.

In bestimmten erfindungsgemäßen Ausführungsformen weist die Hydraulik wenigstens einen Temperatursensor stromabwärts der Druckpumpe oder der Volumenpumpe auf.

In manchen erfindungsgemäßen Ausführungsformen weist die Hydraulik wenigstens eine Vorrichtung zum Überwachen des Drucks (Drucküberwachung) stromabwärts der Volumenpumpe auf.

In bestimmten erfindungsgemäßen Ausführungsformen weist die Hydraulik wenigstens eine Vorrichtung zum Messen des Vordrucks (Vordruckmessung) stromaufwärts der Volumenpumpe auf.

In manchen erfindungsgemäßen Ausführungsformen weist die Hydraulik wenigstens einen Partikelfilter in der Substituatleitung stromaufwärts oder stromabwärts der Volumenpumpe auf.

In bestimmten erfindungsgemäßen Ausführungsformen weist die Hydraulik wenigstens einen Blutdetektor in der Substituatleitung auf.

In einigen erfindungsgemäßen Ausführungsformen wird das mittels der Hydraulik wie hierin beschrieben erzeugte Substituat ohne weitere Maßnahmen, welche einem Dosieren des in den extrakorporalen Blutkreislauf eingebrachten oder einzubringenden Substituats dienen oder dienen könnten, in das in dem extrakorporalen Blutkreislauf vorliegende Blut eingebracht.

In manchen erfindungsgemäßen Ausführungsformen umfasst das Verfahren weiter ein Regeln oder Steuern der Größe des Anteils der Dialysierflüssigkeit, welcher die zweite Filterstufe durchtritt, basierend auf voreingestellten Angaben zum gewünschten Substituatfluss oder Substituatvolumen oder basierend auf während der Behandlung ermittelten Daten, aus welchen in einem Schritt des Verfahrens Angaben über den erforderlichen Substituatfluss errechnet werden. Dabei besteht das Regeln oder Steuern in einem Einwirken auf wenigstens eine Fördervorrichtung und/oder wenigstens eine Flussbegrenzungseinrichtung und/oder ein Flussteilerventil, welche jeweils in der Dialysierflüssigkeitszulaufleitung und/oder der Substituatleitung und/oder in der Abzweigleitung vorliegen oder wirken, oder umfasst ein solches Einwirken.

In einigen erfindungsgemäßen Ausführungsformen weist die Blutbehandlungsvorrichtung eine Steuer- oder Regelvorrichtung auf, welche konfiguriert ist zum Durchführen des Dosierverfahrens.

In einigen erfindungsgemäßen Ausführungsformen der Blutbehandlungsvorrichtung ist die zweite Filterstufe in die Dialysierflüssigkeitszulaufleitung eingefügt und/oder wird von der gesamten dem Dialysator zugeführten Dialysierflüssigkeit durchströmt. Dabei ist in der Dialysierflüssigkeitszulaufleitung stromabwärts der zweiten Filterstufe ein Proportionalventil angeordnet. Ferner ist in der Substituatleitung stromabwärts der zweiten Filterstufe ein Proportionalventil oder eine Drossel angeordnet.

In bestimmten erfindungsgemäßen Ausführungsformen sind die im Zusammenhang mit den hierin beschriebenen erfindungsgemäßen Ausführungsformen genannten Fördereinrichtungen wie eine Pumpe, eine Druckpumpe, eine Flusspumpe oder eine Volumenpumpe oder dergleichen sowie die ebenfalls im Zusammenhang mit den hierin beschriebenen erfindungsgemäßen Ausführungsformen genannten Flussbegrenzungseinrichtungen wie eine Drossel, ein Flussteilerventil, ein Proportionalventil, ein Schlauchquetschventil oder dergleichen konfiguriert, angesteuert, geregelt oder genutzt zum Dosieren des Substituats, insbesondere basierend auf Angaben über den gewünschten Substituatfluss oder das gewünschte Substituatvolumen.

In einigen erfindungsgemäßen Ausführungsformen ist in der Substituatleitung stromabwärts der zweiten Filterstufe eine Druckpumpe angeordnet.

In manchen erfindungsgemäßen Ausführungsformen sind in der Substituatleitung stromabwärts der Druckpumpe ein Temperatursensor und/oder ein Partikelfilter angeordnet.

In einigen erfindungsgemäßen Ausführungsformen geht eine Bypassleitung von der Substituatleitung ab. Diese kann mit der Dialysatablaufleitung verbunden sein.

In bestimmten erfindungsgemäßen Ausführungsformen ist die zweite Filterstufe in die Dialysierflüssigkeitszulaufleitung eingefügt oder wird von der Dialysierflüssigkeit durchströmt (d. h. sie ist entsprechend angeordnet). Dabei ist in der Substituatleitung stromabwärts der zweiten Filterstufe eine Volumenpumpe angeordnet.

In manchen erfindungsgemäßen Ausführungsformen ist in der Substituatleitung stromaufwärts der Volumenpumpe ein Substituatdrucksensor angeordnet. In einigen erfindungsgemäßen Ausführungsformen ist stromabwärts der Volumenpumpe ein Partikelfilter und/oder ein Drucksensor angeordnet.

In bestimmten erfindungsgemäßen Ausführungsformen ist in der Dialysierflüssigkeitszulaufleitung und/oder in der Substituatleitung wenigstens ein Flusssensor angeordnet.

In manchen erfindungsgemäßen Ausführungsformen geht von der Dialysierflüssigkeitszulaufleitung wie oben beschrieben an einem Abzweigungspunkt eine Abzweigleitung ab, welche stromabwärts des Abzweigungspunkts in die zweite Filterstufe leitet, mündet oder dieser Fluid zuführt, wobei die Substituatleitung aus der zweiten Filterstufe - insbesondere direkt oder indirekt - hervorgeht. In diesen Ausführungsformen liegt die Substituatleitung stromabwärts der zweiten Filterstufe. Die Substituatleitung beginnt nicht schon vor oder stromaufwärts der zweiten Filterstufe. Die zweite Filterstufe wird nicht von jenem Anteil der Dialysierflüssigkeit durchströmt, welcher dem Dialysator zugeführt wird.

In einigen erfindungsgemäßen Ausführungsformen ist im Abzweigungspunkt und damit stromaufwärts der zweiten Filterstufe ein Flussteilerventil angeordnet.

In bestimmten erfindungsgemäßen Ausführungsformen ist in der Dialysierflüssigkeitszulaufleitung stromabwärts des Abzweigungspunkts wenigstens ein Proportionalventil angeordnet.

In manchen erfindungsgemäßen Ausführungsformen ist in der Abzweigleitung stromaufwärts der zweiten Filterstufe wenigstens ein Proportionalventil oder wenigstens eine Drossel angeordnet.

In bestimmten erfindungsgemäßen Ausführungsformen ist in der Abzweigleitung stromabwärts des Abzweigungspunkts eine Vordruckpumpe angeordnet.

In manchen erfindungsgemäßen Ausführungsformen ist in der Abzweigleitung stromabwärts einer in der Abzweigleitung gelegenen Vordruckpumpe ein Temperatursensor angeordnet.

In bestimmten erfindungsgemäßen Ausführungsformen ist in der Abzweigleitung stromabwärts des Abzweigungspunkts eine Volumenpumpe angeordnet.

In manchen erfindungsgemäßen Ausführungsformen ist in der Abzweigleitung stromabwärts der Volumenpumpe, aber stromaufwärts der zweiten Filterstufe ein Abzweigungsdrucksensor vorgesehen.

In bestimmten erfindungsgemäßen Ausführungsformen ist in der Abzweigleitung stromaufwärts des Abzweigungspunkts eine Vordruckpumpe angeordnet.

In manchen erfindungsgemäßen Ausführungsformen geht von der zweiten Filterstufe eine Flushleitung ab.

In bestimmten erfindungsgemäßen Ausführungsformen ist in der Substituatleitung und/oder in der Dialysierflüssigkeitszulaufleitung stromabwärts des Abzweigungspunkts und/oder stromabwärts der zweiten Filterstufe und/oder in der Abzweigleitung wenigstens ein Flusssensor angeordnet.

In bestimmten erfindungsgemäßen Ausführungsformen ist die Blutbehandlungsvorrichtung als Hämodialysevorrichtung, Hämofiltrationsvorrichtung oder Hämodiafiltrationsvorrichtung ausgestaltet.

In einigen erfindungsgemäßen Ausführungsformen ist die medizinische Funktionsvorrichtung ausgestaltet als Blutkassette oder als extrakorporaler Blutschlauch oder Blutschlauchsatz.

In manchen erfindungsgemäßen Ausführungsformen ist die medizinische Funktionsvorrichtung ein Einweg- oder Wegwerfartikel.

In bestimmten erfindungsgemäßen Ausführungsformen gilt das zum "Dosieren" Gesagte auch für ein "Erzeugen" oder "Bereitstellen".

In einigen erfindungsgemäßen Ausführungsformen ist das Einbringen des Substituats in den extrakorporalen Kreislauf nicht Teil des Verfahrens.

In bestimmten erfindungsgemäßen Ausführungsformen weisen die erfindungsgemäßen Vorrichtungen die zum Ausführen des Verfahrens erforderlichen Einrichtungen, Bauteile und Komponenten auf, insbesondere Ventile, Fördereinrichtungen wie beispielsweise Pumpen, Leitungen, Regeleinrichtungen, Steuereinrichtungen, usw.

Wo immer hierin von einem Sensor wie einem Druck-, Temperatur- oder Flusssensor die Rede ist, wird erfindungsgemäß jede Vorrichtung verstanden, welche zum Ermitteln oder Messen des jeweiligen Parameters geeignet und eingesetzt wird.

Wo immer hierin von einer Substituatleitung oder einem Substituat die Rede ist, so kann erfindungsgemäß hierunter auch eine Leitungsanordnung zum Erzeugen einer anderen, insbesondere hochreinen, Flüssigkeit verstanden werden. Diese kann beispielsweise als Spülflüssigkeit zum Reinigen oder zur Desorption von beladenen Adsorberkartuschen (über welche beispielsweise im Rahmen einer Blutbehandlung Blutplasma geleitet wird) vorgesehen oder verwendet werden. Derartige Spülflüssigkeiten, welche ebenfalls wie hierin beschrieben erzeugt werden können, werden üblicherweise nicht als Substituate bezeichnet. Aufgrund des Vorstehenden soll die vorliegende Erfindung daher nicht auf Substituat beschränkt sein. Jede andere wie hierin beschrieben hergestellte oder dosierte Flüssigkeit fällt - wie die hierfür verwendeten Verfahren und Vorrichtungen - ebenfalls unter die vorliegende Erfindung. Die vorliegende Erfindung kann daher beispielsweise auch verwendet werden, um eine für den Blutkontakt geeignete Spülflüssigkeit für die Abreinigung oder Desorption von beladenen Adsorberkartuschen zu dosieren.

Manche oder alle erfindungsgemäßen Ausführungsformen können einen, mehrere oder alle der oben und/oder im Folgenden genannten Vorteile aufweisen.

Ein Vorteil kann darin bestehen, dass in Ausgestaltungen, in denen neben der vorliegenden Erfindung auch noch eine herkömmliche Dosiervorrichtung, wie sie aus dem Stand der Technik bekannt und einleitend erwähnt ist, zum Einsatz kommt, aufgrund der bereits durch die erfindungsgemäße Hydraulik erfolgenden Dosierung des Substituats die Prozesssicherheit erhöht werden kann. Fehler der herkömmlichen Dosiervorrichtung können nicht zu einer Überdosierung an Substituat führen.

Ein weiterer Vorteil kann darin bestehen, dass eine Dosiereinrichtung bei erfindungsgemäßen Vorrichtungen oder deren Einsatz nicht mehr vorgesehen sein muss. Die erforderliche Dosierung erfolgt in der gewünschten Genauigkeit bereits durch die Hydraulik der Blutbehandlungsvorrichtung gemäß der vorliegenden Erfindung. Dies spart die Kosten, welche für das Vorsehen einer eigens zum Dosieren des Substituats vorgesehenen Dosiereinrichtung angesetzt werden müssen. Ferner kann dies den Aufwand für Montage, Kalibrierung, Überwachung und Wartung der erfindungsgemäß nicht mehr erforderlichen Dosiervorrichtung sowie die hierbei jeweils anfallenden Kosten einsparen helfen.

Durch die Verlagerung des Schritts der Dosierung des Substituats auf die Blutbehandlungsvorrichtung kann die Dosiergenauigkeit ferner auf verlässlichere Weise sichergestellt werden, als dies mit Lösungen des Standes der Technik, bei welchen die Dosierung teilweise oder ganz auf Seiten eines Blutschlauchsatzes oder einer Blutkassette, also Einwegartikeln, verlagert ist.

Ein weiterer Vorteil kann darin bestehen, dass mittels der vorliegenden Erfindung mit einer hohen Genauigkeit oder gar mit einer höheren Genauigkeit als bislang gefördert werden kann, was das geförderte Volumen betrifft. Ein Grund hierfür kann darin liegen, dass einige der Toleranzen, welche jede für sich die Genauigkeit mindern können, wegfallen können. Dies trifft beispielsweise auf die Compliance des Substituatschlauchs und/oder auf die Federkraft der Rollenpumpe zu.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil stark vereinfachten Figuren gilt:
- **Fig. 1**: zeigt schematisch vereinfacht und in Ausschnitten die Hydraulik der Blutbehandlungsvorrichtung in einer ersten erfindungsgemäßen Ausführungsform;
- **Fig. 2**: zeigt schematisch vereinfacht und in Ausschnitten die Hydraulik der Blutbehandlungsvorrichtung in einer zweiten erfindungsgemäßen Ausführungsform;
- **Fig. 3**: zeigt schematisch vereinfacht und in Ausschnitten die Hydraulik der Blutbehandlungsvorrichtung in einer dritten erfindungsgemäßen Ausführungsform;
- **Fig. 4**: zeigt schematisch vereinfacht und in Ausschnitten die Hydraulik der Blutbehandlungsvorrichtung in einer vierten erfindungsgemäßen Ausführungsform;
- **Fig. 5**: zeigt schematisch vereinfacht und in Ausschnitten die Hydraulik der Blutbehandlungsvorrichtung in einer fünften erfindungsgemäßen Ausführungsform;
- **Fig. 6**: zeigt schematisch vereinfacht und in Ausschnitten die Hydraulik der Blutbehandlungsvorrichtung in einer sechsten erfindungsgemäßen Ausführungsform;
- **Fig. 7**: zeigt schematisch vereinfacht und in Ausschnitten die Hydraulik der Blutbehandlungsvorrichtung in einer siebten erfindungsgemäßen Ausführungsform; und
- **Fig. 8**: zeigt schematisch vereinfacht und in Ausschnitten die Hydraulik der Blutbehandlungsvorrichtung in einer achten erfindungsgemäßen Ausführungsform.

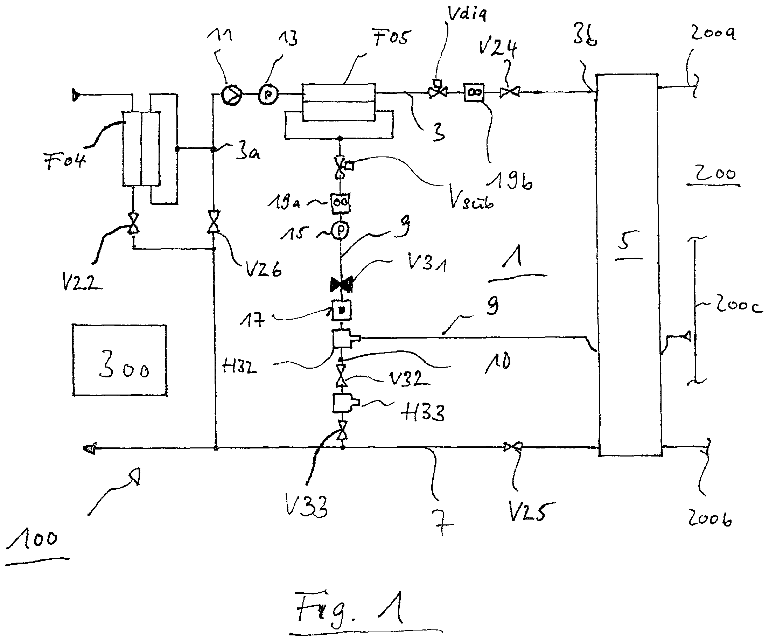

**Fig. 1** zeigt schematisch vereinfacht die erfindungsgemäße Hydraulik 1 einer erfindungsgemäßen Behandlungsvorrichtung 100, mittels welcher das Verfahren durchführbar ist, und einen nur schematisch angedeuteten Blutkreislauf 200 als Beispiel einer medizinischen Funktionsvorrichtung.

Die Hydraulik 1 weist eine Dialysierflüssigkeitszulaufleitung 3, auch als "Dialysatleitung" bezeichnet, auf, welche online, d. h. mittels der Behandlungsvorrichtung 100 erzeugte Dialysierflüssigkeit zu einem Blutfilter oder Dialysator 5 führt. Als Dialysierflüssigkeitszulaufleitung 3 wird hierin die gesamte durchströmte Leitung verstanden, welche sich von einer Einmündung 3a stromabwärts eines auch als die erste Filterstufe bezeichneten ersten Filters F04 bis zum Eintritt der Dialysierflüssigkeitszulaufleitung 3 an einer Eintrittsstelle 3b in den Dialysator 5 erstreckt.

An den Dialysator 5 schließt sich eine Dialysatablaufleitung 7 an, welche die Dialysierflüssigkeit aus dem Dialysator 5 abführt. Die mittels der Dialysierflüssigkeitszulaufleitung 3 dem Dialysator 5 zugeführte Dialysierflüssigkeit passiert vor ihrem Eintritt in den Dialysator 5 zusätzlich zum ersten Filter F04 ferner einen auch als zweite Filterstufe bezeichneten zweiten Filter F05. Der zweite Filter F05 ist in die Dialysierflüssigkeitszulaufleitung 3 eingefügt und wird von der Dialysierflüssigkeit durchströmt. Dialysat kann den zweiten Filter F05 längs der Dialysierflüssigkeitszulaufleitung 3 durchströmen, ohne gefiltert zu werden.

Im zweiten Filter F05 wird dadurch im Folgenden auch als "Substituat" bezeichnetes Filtrat erzeugt, dass als ein Anteil der Dialysierflüssigkeit durch die Membran oder Sterilmembran des zweiten Filters F05 filtriert, hindurchgeführt oder hindurchgepresst und in eine Substituatleitung 9 geführt wird. Aus der Substituatleitung 9 kann das auf diese Weise erzeugte Substituat beispielsweise über einen Substituatport H32 einem extrakorporalen Blutkreislauf 200, welcher teilweise auf einer nicht dargestellten Blutkassette verlaufen kann, zugeführt werden. Dies kann optional in Prädilution und/oder Postdilution erfolgen. Der extrakorporale Blutkreislauf 200, welcher in den hier anhängenden Figuren nur schematisch angedeutet ist, weist wenigstens eine Blutablaufleitung 200a auf, welche mit dem Dialysator 5 verbunden ist, eine Blutzulaufleitung 200b, welche ebenfalls mit dem Dialysator 5 verbunden ist, und einen Abschnitt 200c, welcher in direkter Fluidverbindung mit der Substituatleitung 9 steht.

In die Dialysierflüssigkeitszulaufleitung 3 ist optional eine Klemme oder ein Ventil V24 eingefügt. In die Dialysatablaufleitung 7 ist optional eine Klemme oder ein Ventil V25 eingefügt. In die Substituatleitung 9 ist zwischen dem zweiten Filter F05 und dem Substituatport H32 optional eine Klemme oder ein Ventil V31 eingefügt.

An den Substituatport H32 schließt sich eine Verbindungsleitung 10 an. Sie verbindet die Substituatleitung 9 mit der Dialysatablaufleitung 7. Weitere, ebenfalls nur optional vorgesehene Ventile V32 und V33 sind in Fig. 1 in der Verbindungsleitung 10 gezeigt. Ebenfalls optional sind ferner ein Retentionsventil V22, ein Bypassventil V26 und ein Spülport H33 vorgesehen.

Zwischen dem ersten Filter F04 und dem zweiten Filter F05 können, jeweils optional, eine Vordruckpumpe 11 und ein Dialysatvordrucksensor 13 vorgesehen sein. Ebenso können in der Substituatleitung 9 ein Substituatdrucksensor 15 und ein Blutdetektor 17, jeweils optional, vorgesehen sein.

Von einem "Vordruck" ist erfindungsgemäß dann die Rede, wenn das betreffende Bauteil - wie etwa die Vordruckpumpe 11 oder der Dialysatvordrucksensor 13 - stromaufwärts des zweiten Filters F05 angeordnet ist oder wirkt.

Zum Dosieren des Substituatflusses oder -volumens sind in der in Fig. 1 dargestellten ersten Ausführungsform ein erstes Proportionalventil Vdia, welches in die Dialysierflüssigkeitszulaufleitung 3 eingefügt ist, und ein zweites Proportionalventil Vsub, welches in die Substituatleitung 9 eingefügt ist, vorgesehen. Es sei angemerkt, dass es sich bei den Elementen Vdia und Vsub nur beispielsweise um Proportionalventile handelt. Sie können auch auf andere, dem Fachmann bekannte Weise als geeignete Fluss- oder Strömungsbegrenzungseinrichtungen ausgestaltet sein. Die Ventilstellung oder Ventilstellungen werden in der in Fig. 1 gezeigten Ausführungsform derart gesteuert oder geregelt, dass die gewünschte Flussteilung zwischen dem Dialysatflüssigkeitsstrom in der Dialysierflüssigkeitszulaufleitung 3 und dem Substituatstrom in der Substituatleitung 9 erzielt wird.

Für den Fall, dass sichergestellt werden können soll, dass der Dialysatvordruck, welcher beispielsweise mit dem Dialysatvordrucksensor 13 gemessen werden kann, nicht unter einen definierten oder vorbestimmten Druckwert abfällt, kann die Ventilstellung eines der beiden Proportionalventile Vdia und Vsub oder die Ventilstellungen beider Proportionalventile Vdia und Vsub entsprechend eingestellt oder geregelt werden. Dieser vorbestimmte Druckwert kann derart bestimmt werden, dass sowohl in der Dialysierflüssigkeitszulaufleitung 3 als auch in der Substituatleitung 9 jeweils definierte Flüsse sichergestellt werden können. Eine optional vorgesehene obere Druckbegrenzung kann durch die Hydraulik erfolgen. Bei Unterschreiten eines Mindestdrucks kann als Sicherheitsmaßnahme optional das Schließen der Substituatleitung 9, beispielsweise mittels des Ventils V31, erfolgen.

Alternativ oder ergänzend kann ein gewünschter Vordruck durch eine Pumpe, beispielsweise durch die optional vorhandene Vordruckpumpe 11, erzeugt oder sichergestellt werden.

Optional sind zur Überwachung der erzielten oder der gewünschten Flussteilung ferner in der Substituatzulaufleitung 3 ein Flusssensor 19a und/oder in der Substituatleitung 9 ein Flusssensor 19b vorgesehen. Dabei liegt der Flusssensor 19a stromabwärts des Ventils Vsub, der Flusssensor 19b liegt stromabwärts des Ventils Vdia. Es wird angemerkt, dass entgegen der hier beschriebenen, exemplarischen Ausführungsform erfindungsgemäß einer, manche oder alle der Flusssensoren ungeachtet der Lage der übrigen Flusssensoren alternativ auch stromaufwärts der Proportionalventile liegen können, solange sie stromabwärts der Abzweigstelle von Dialysat und Substituat oder dem Abzweigpunkt der Abzweigleitung liegen.

Die gewünschte Flussteilung kann optional mittels entsprechender Druckmessungen und der hierzu optional vorgesehenen Druckmessvorrichtungen überwacht und sichergestellt werden. Für diesen Fall kann es vorteilhaft möglich sein, auf die optional vorhandenen Flusssensoren 19a und 19b zu verzichten.

Wird, wie in weiteren erfindungsgemäßen Ausführungsformen der vorliegenden Erfindung vorgesehen, das Ventil Vsub als Schlauchquetschventil ausgeführt, so kann darauf verzichtet werden, in der Substituatleitung 9 auch ein Ventil V31 vorzusehen. In einem solchen Fall kann vorteilhaft ein Flusssensor eingesetzt werden. Mit ihm kann eine gewünschte Förderratengenauigkeit von beispielsweise 10% leicht überprüft und ggf. entsprechend nachgeregelt werden.

Die zur Fig. 1 gemachten Erläuterungen treffen, wo für den Fachmann als sinnvoll erkannt, auch auf die folgenden Figuren zu. Dies gilt insbesondere für die in Fig. 1 gezeigten Bauteile, ihre Bezeichnungen und ihre Funktionen.

Zum Steuern oder Regeln der oben genannten Komponenten der Hydraulik 1 zum Ausführen des Verfahrens weist die Blutbehandlungsvorrichtung 100 eine Regel- oder Steuervorrichtung 300 auf oder ist hiermit in Signal- oder Wirkverbindung verbunden.

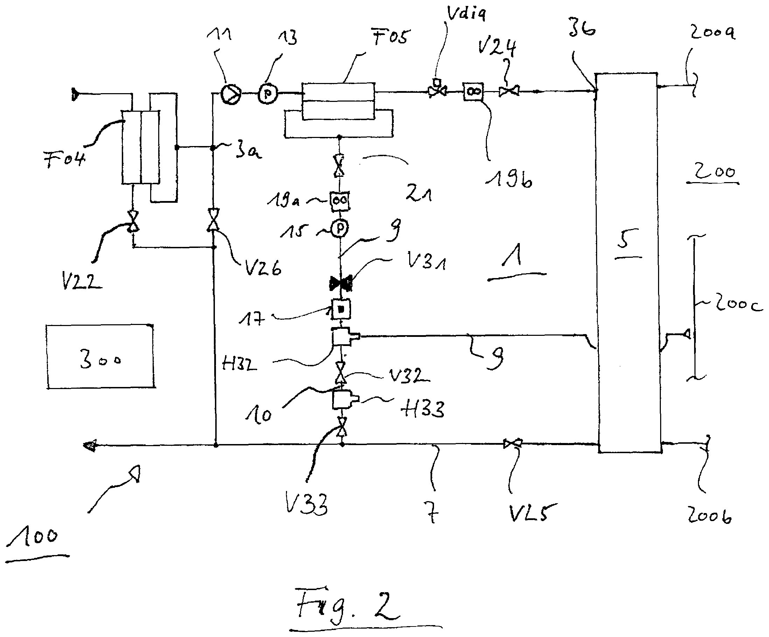

**Fig. 2** zeigt wiederum schematisch vereinfacht und nur in Ausschnitten die Hydraulik 1 der Blutbehandlungsvorrichtung 100 in einer zweiten erfindungsgemäßen Ausführungsform. In dem in Fig. 2 gezeigten Aufbau ist anstelle des optional als Proportionalventil ausgestalteten Ventils Vsub an der Stelle, an welcher das Ventil Vsub in Fig. 1 gezeigt ist, wiederum rein optional eine Drossel 21 vorgesehen. Im Übrigen kann der Aufbau der Fig. 2 jenem der Fig. 1 entsprechen.

Die mit Bezug auf Fig. 2 offenbarte erfindungsgemäße Ausführungsform, in welcher nur ein Proportionalventil, nämlich das Ventil Vdia vorgesehen ist, bietet sich vor allem dann an, wenn sichergestellt werden kann, dass der Druckabfall über der Substituatleitung 9 oder dem gesamten Substituatzweig stets höher ist als über der Dialysierflüssigkeitszulaufleitung 3 oder dem gesamten Dialysatzweig. Ist dies der Fall, was bei einer Hämodiafiltrationsbehandlung stets anzunehmen ist, da andernfalls kein Dialysat mehr fließen würde und die Behandlung zu einer Hämofiltrationsbehandlung werden würde, lässt sich wie in Fig. 2 gezeigt ein Proportionalventil einsparen.

Es wird davon ausgegangen, dass der Druckabfall über dem Substituatzweig in aller Regel höher sein sollte als über dem Dialysatzweig, da die in der Dialysierflüssigkeitszulaufleitung verbleibende Dialysierflüssigkeit den zweiten Filter F05 longitudinal passiert, der in den Substituatzweig abgeführte Teil der Dialysierflüssigkeit jedoch durch die Membran des zweiten Filters F05 hindurch gepresst werden muss.

Ferner befindet sich im Substituatzweig in aller Regel ein auf einem Disposable wie einer Blutkassette oder dem extrakorporalen Blutkreislauf vorliegendes, hier nicht gezeigtes Rückschlagventil, zum Verhindern eines Rückstroms. Dieses Rückschlagventil weist einen Öffnungsdruck auf, um die Sperrfunktion des Rückschlagventils sicherzustellen. Daher ist der Druckabfall über dem Substituatzweig höher. Der Öffnungsdruck kann exemplarisch mehr als 100 mbar betragen.

Soll sichergestellt werden, dass der Druckabfall über dem Substituatzweig höher ist als der Druckabfall über dem Dialysierflüssigkeitszweig, so kann der Substituatzweig wie in Fig. 2 gezeigt mit einer Drossel 21 versehen werden. Bei deren Auslegung kann der maximal zulässige Substituatstrom sowie der maximale oder maximal zulässige Dialysatvordruck berücksichtigt werden.

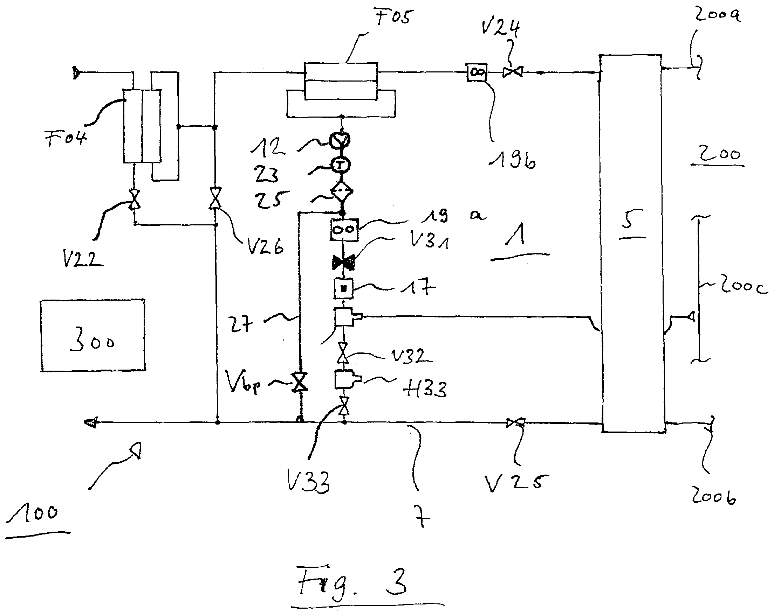

**Fig. 3** zeigt eine dritte erfindungsgemäße Ausführungsform. Die Erläuterungen hierzu entsprechen im Wesentlichen jenen zu den Fig. 1 und 2. Gegenüber den Darstellungen der Fig. 1 und 2 ist jedoch anstelle der stromaufwärts des zweiten Filters F05 liegenden Vordruckpumpe 11 eine stromabwärts des zweiten Filters F05 liegende Pumpe 12 in der Substituatleitung 9 angeordnet. Zudem können optional stromabwärts der beispielsweise als Druckpumpe ausgestalteten Pumpe 12 ein Temperatursensor 23 und/oder ein Partikelfilter 25 vorgesehen sein. Basierend auf den vom Temperatursensor 23 gelieferten Temperaturwerten kann sichergestellt werden, dass das dem Blutkreislauf 200 zugeführte Substituat nicht unzulässig stark erwärmt wurde, was durch die Wirkung der vorgelagerten Pumpe 12 erfolgt sein könnte. Sollte eine unzulässig starke Erwärmung festgestellt werden, so kann das erwärmte Substituat ganz oder teilweise mittels einer optional vorgesehenen Bypassleitung 27 durch Öffnen eines angeordneten Bypassventils Vbp abgeleitet werden.

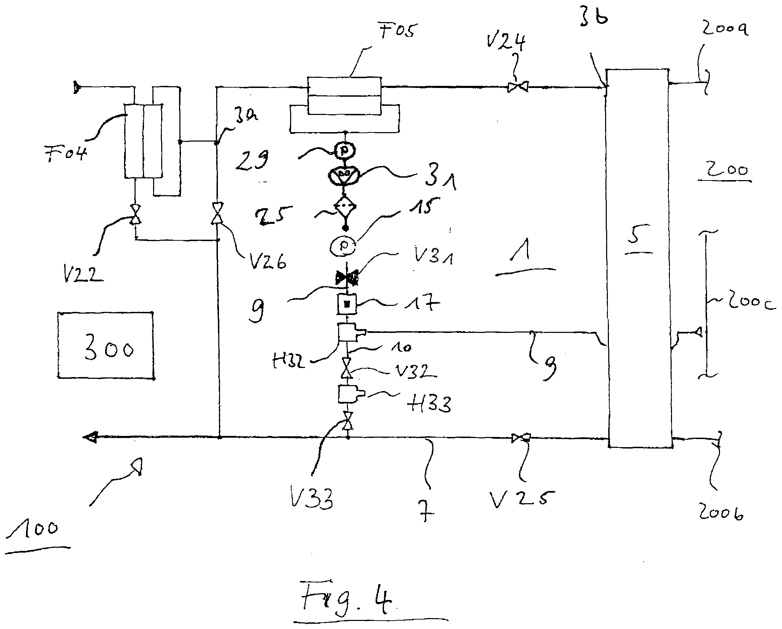

**Fig. 4** zeigt wiederum schematisch vereinfacht und nur in Ausschnitten die Hydraulik 1 der Blutbehandlungsvorrichtung 100 in einer vierten erfindungsgemäßen Ausführungsform.

In der Ausführungsform der Fig. 4 ist stromabwärts des zweiten Filters F05 in der Substituatleitung 9 optional ein Substituatvordrucksensor 29 vorgesehen. Anstelle einer in der Dialysierflüssigkeitszulaufleitung 3 vorgesehenen Vordruckpumpe 11 ist bei der Ausführungsform der Fig. 4 in der Substituatleitung 9 eine Volumenpumpe 31 vorgesehen. Diese liegt hier stromabwärts des zweiten Filters F05 und - falls vorhanden - stromabwärts des Substituatvordrucksensors 29.

Auch in der in Fig. 4 gezeigten Ausführungsform kann optional ein Partikelfilter 25 vorgesehen sein. Dieser kann stromabwärts der Volumenpumpe 31 angeordnet sein.

Zudem weist die Substituatleitung 9 einen Substituatsensor 15 auf. Dieser liegt stromabwärts der Volumenpumpe.

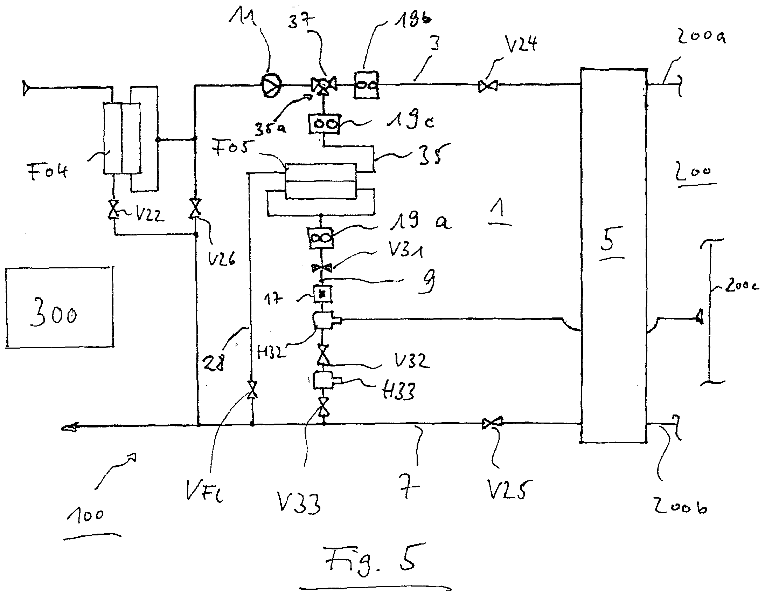

Die im Folgenden diskutierten Fig. 5 bis 8 zeigen weitere erfindungsgemäße Ausführungsformen, welche sich von jenen der Fig. 1 bis 4 darin unterscheiden, dass der zweite Filter F05 nicht Teil der Dialysierflüssigkeitszulaufleitung 3 ist. Tatsächlich wird der zweite Filter F05 anders als zu den Fig. 1 bis 4 diskutiert nicht auch von jener Dialysierflüssigkeit durchströmt, welche in den Dialysator 5 eintritt. Der zweite Filter F05 wird in den Anordnungen der Fig. 5 bis 8 allein von jenem Anteil der online mittels des ersten Filters F04 erzeugten Dialysierflüssigkeit durchströmt, welcher zu Erzeugung von Filtrat oder Substituat verwendet wird.

Dies erfolgt in den Anordnungen der Fig. 5 bis 8 dadurch, dass zwischen der Dialysierflüssigkeitszulaufleitung 3 und dem zweiten Filter F05 eine an einem Abzweigungspunkt 35a beginnende Abzweigleitung 35 vorgesehen ist.

**Fig. 5** zeigt wiederum schematisch vereinfacht und nur in Ausschnitten die Hydraulik 1 der Blutbehandlungsvorrichtung 100 in jeweils einer fünften erfindungsgemäßen Ausführungsform.

Anders als in den vorangegangenen Figuren dargestellt sorgt ein am Abzweigungspunkt 35a vorgesehenes Flussteilerventil 37 dafür, dass der den ersten Filter F04 durchströmende Volumenfluss, welcher optional durch eine Vordruckpumpe 11 gefördert wird, im gewünschten Verhältnis in einen Dialysatstrom und in einen Abzweig- oder Substituatstrom aufgeteilt wird. Mittels integrierter Druckwaagen kann dieses Flussverhältnis unabhängig vom jeweiligen Gegendruck aufrechterhalten werden. Zur Sicherstellung der Funktion kann die vor dem Flussteilerventil 37 optional vorgesehene Vordruckpumpe 11 den zum Betrieb des Flussteilerventils 37 erforderlichen Vordruck bereitstellen. Die Anordnung der Fig. 5 weist eine Flushleitung 28 auf. Diese enthält ein Spülventil VF1.

Anders als die vorangegangenen Figuren weist die Fig. 5 einen dritten, nur optional vorgesehenen Flusssensor 19c auf, welcher in der Abzweigleitung 35 angeordnet ist. Der dritte Flusssensor 19c kann optional zum ersten Flusssensor 19a oder zum zweiten Flusssensor 19b oder zu beiden Flusssensoren 19a und 19b vorgesehen sein. Erfindungsgemäß genügt es auch, nur einen der Flusssensoren 19a, 19b und 19c vorzusehen oder beliebige Kombinationen hiervon, beispielsweise an den in Fig. 5 gezeigten Stellen der Hydraulik 1.

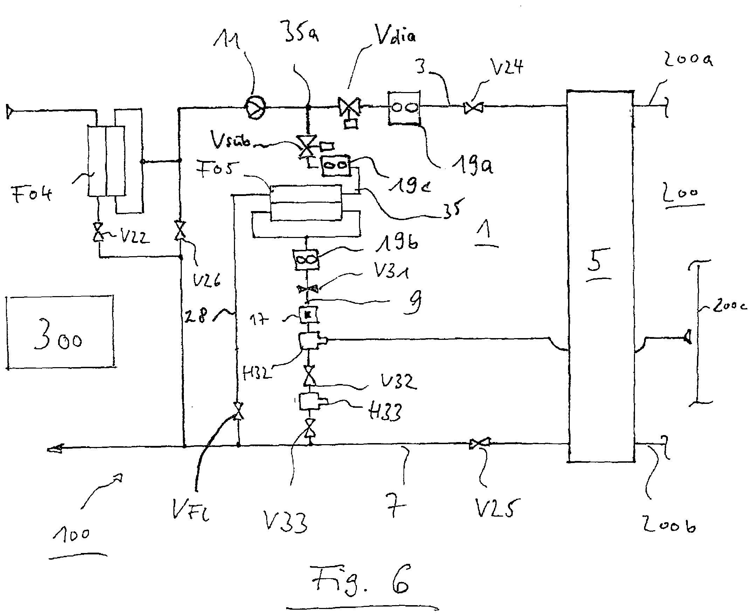

**Fig. 6** zeigt in Anlehnung der Fig. 5 wiederum schematisch vereinfacht die Hydraulik 1 in einer sechsten erfindungsgemäßen Ausführungsform.

Die Flussteilung wird in Fig. 6 wiederum mit zwei Proportionalventilen Vdia und Vsub wie dargestellt nachgebildet. Die hiermit einhergehenden Vorteile umfassen die verringerte mechanische Komplexität und eine verbesserte Reinigungsmöglichkeit durch Personal und/oder Maschine.

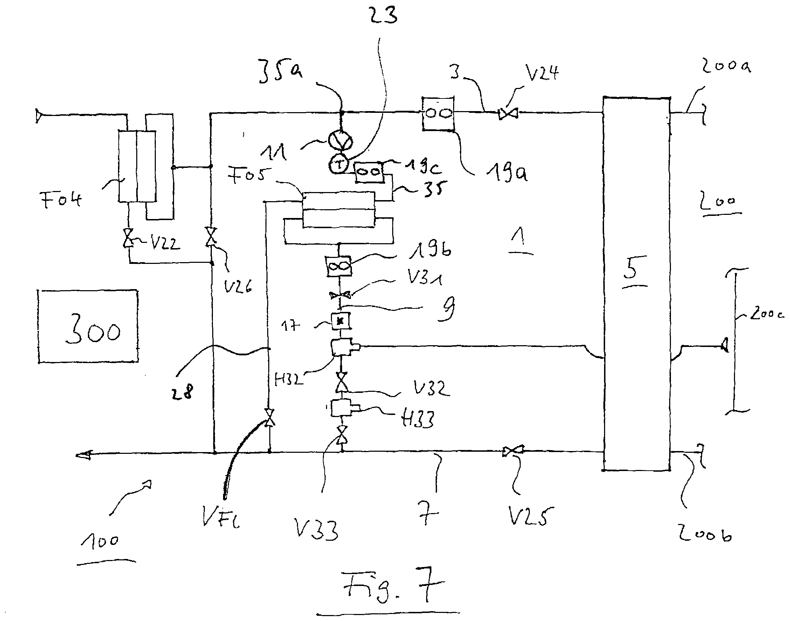

**Fig. 7** zeigt eine siebte erfindungsgemäße Ausführungsform. Bei ihr ist - vorzugsweise in der Abzweigleitung 35 eine Vordruckpumpe 11 vorgesehen, welche mit Hilfe eines oder mehrerer der Flusssensoren 19a, 19b und 19c zum Erzielen des gewünschten Substituatflusses geregelt werden kann. Die Druckpumpe oder Vordruckpumpe 11 kann beispielsweise eine Zahnradpumpe mit Bypass oder eine Zentrifugalpumpe sein.

Da beispielsweise eine Zentrifugalpumpe das Substituat merklich erwärmen kann, kann optional zur Überwachung von dessen Temperatur ein Temperatursensor 23 stromabwärts der Vordruckpumpe 11 vorgesehen sein. Erfindungsgemäß kann vorgesehen sein, dass bei Erkennen einer Temperaturüberschreitung oder bei Überschreiten eines vorbestimmten Temperaturgrenzwertes das zu warme Substituat über das Spülventil VF1 und die Flushleitung 28 abgeführt wird. Zu diesem Zweck kann das Ventil V31 ganz oder teilweise geschlossen werden.

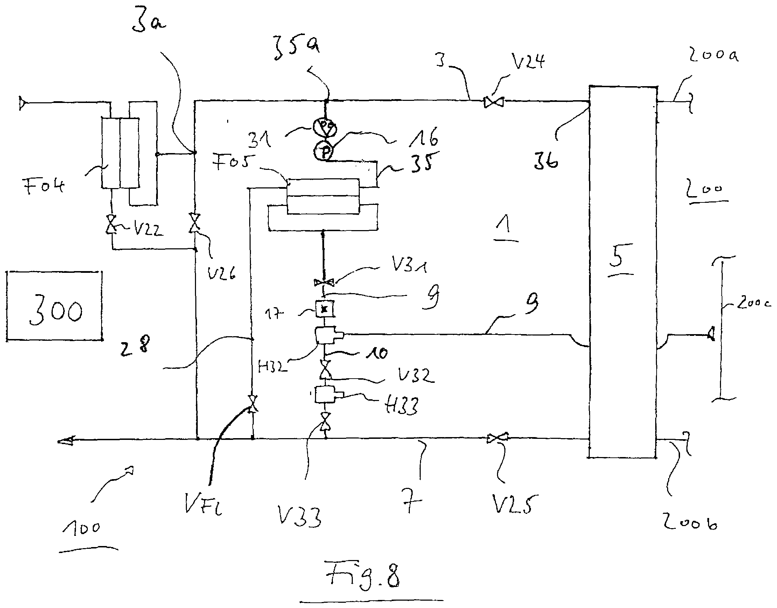

**Fig. 8** zeigt die erfindungsgemäße Hydraulik 1 einer erfindungsgemäßen Behandlungsvorrichtung 200 gemäß einer achten erfindungsgemäßen Ausführungsform.

Bei dieser Ausgestaltung ist eine Volumenpumpe 31 - vorzugsweise in der Abzweigungsleitung 35 - vorgesehen. Sie kann einen definierten Substituatfluss erzeugen. Die Volumenpumpe oder Flusspumpe 31 kann beispielsweise als eine Zahnradpumpe ohne Bypass, eine Membranpumpe, eine Schlauchrollenpumpe oder auch als eine Drehschieberpumpe ausgestaltet sein.

Bei dieser oder vergleichbaren Ausführungsformen gemäß der vorliegenden Erfindung kann zur Begrenzung des Drucks in der Abzweigleitung 35 je nach verwendetem Pumpentyp eine Drucküberwachung mittels einer geeigneten Druckmessvorrichtung wie beispielsweise dem Abzweigleitungsdrucksensor 16 vorgesehen sein.

Einige der Merkmale der in den Figuren dargestellten erfindungsgemäßen Ausführungsformen gehen aus der folgenden Tabelle hervor.

| Merkmal | Fig. 1 | Fig. 2 | Fig. 3 | Fig. 4 | Fig. 5 | Fig. 6 | Fig. 7 | Fig. 8 |
|---|---|---|---|---|---|---|---|---|
| | | | | | | | | |
| herkömmliche Schaltung der zweiten Filterstufe in Dialysierflüssigkeitszulaufleitung | ja | ja | ja | ja | nein | nein | nein | nein |
| | | | | | | | | |
| zweite Filterstufe in Abzweigleitung der Dialysierflüssigkeitszulaufleitung | nein | nein | nein | nein | ja | ja | ja | ja |
| | | | | | | | | |
| Flushleitung am Ausgang der Dialysatkammer der zweiten Filterstufe | nein | nein | nein | nein | ja | ja | ja | ja |
| | | | | | | | | |
| Bypassleitung abgehend von der Filtratleitung zum Flush | nein | nein | ja | nein | nein | nein | nein | nein |
| | | | | | | | | |
| Flussteilerventil in Dialysierflüssigkeitszulaufleitung nach erster Filterstufe | nein | nein | nein | nein | ja | nein | nein | nein |
| | | | | | | | | |
| Proportionalventil in Dialysierflüssigkeitszulaufleitung nach zweiter Filterstufe | ja (opt.) | ja | nein | nein | nein | ja (opt.) | nein | nein |
| | | | | | | | | |
| Proportionalventil in Substituatleitung nach zweiter Filterstufe | ja (opt.) | nein | nein | nein | nein | nein | nein | nein |
| | | | | | | | | |
| Flussmessung in Dialysierflüssigkeitszulaufleitung | ja (opt.) | ja (opt.) | ja (opt.) | nein | ja (opt.) | ja (opt.) | ja (opt.) | nein |
| | | | | | | | | |
| Flussmessung in Abzweigleitung vor der zweiten Filterstufe | nein | nein | nein | nein | ja (opt.) | ja (opt.) | ja (opt.) | nein |
| | | | | | | | | |
| Flussmessung in Substituatleitung nach der zweiten Filterstufe | ja | ja (opt.) | ja (opt.) | nein | ja (opt.) | ja (opt.) | ja (opt.) | nein |
| Vordruckpumpe in Dialysierflüssigkeitszulaufleitung nach der ersten Filterstufe | ja | ja | nein | nein | ja | ja | nein | nein |
| | | | | | | | | |
| Vordruckmessung in Dialysierflüssigkeitszulaufleitung nach der ersten Filterstufe | ja | ja | nein | nein | nein | nein | nein | nein |
| | | | | | | | | |
| Vordruckpumpe in Abzweigleitung vor der zweiten Filterstufe | nein | nein | nein | nein | nein | nein | ja | nein |
| | | | | | | | | |
| Druckpumpe in Substituatleitung nach der zweiten Filterstufe | nein | nein | ja | nein | nein | nein | nein | nein |
| | | | | | | | | |
| Volumenpumpe in Abzweigleitung vor der zweiten Filterstufe | nein | nein | nein | nein | nein | nein | nein | ja |
| | | | | | | | | |
| Volumenpumpe in Abzweigleitung nach der zweiten Filterstufe | nein | nein | nein | ja | nein | nein | nein | nein |
| | | | | | | | | |
| Temperatursensor nach Druckpumpe | nein | nein | ja | nein | nein | nein | ja | nein |
| | | | | | | | | |
| Drucküberwachung nach Volumenpumpe | nein | nein | nein | ja | nein | nein | nein | ja |
| | | | | | | | | |
| Vordruckmessung vor Volumenpumpe | nein | nein | nein | ja | nein | nein | nein | nein |
| | | | | | | | | |
| Partikelfilter in Substituatleitung nach Volumenpumpe | nein | nein | nein | ja | nein | nein | nein | nein |
| | | | | | | | | |
| Blutdetektor in Substituatleitung (generell optional) | ja | ja | ja | ja | ja | ja | ja | ja |

## Patentansprüche

1. Blutbehandlungsvorrichtung (100) mit
- einer Hydraulik (1) mit wenigstens einer ersten Filterstufe (F04) und einer zweiten Filterstufe (F05), mit wenigstens einer Fördervorrichtung (11, 12, 31), wenigstens einer Flussbegrenzungseinrichtung (Vdia, Vsub, 21) oder einem Flussteilerventil (37), sowie mit wenigstens einer Dialysierflüssigkeitszulaufleitung (3) und wenigstens einer Substituatleitung (9), sowie optional wenigstens einer die Dialysierflüssigkeitszulaufleitung (3) und die Substituatleitung (9) verbindenden Abzweigleitung (35);
- einer medizinischen Funktionsvorrichtung, welche ausgestaltet ist als Blutkassette, und welche eine Substituatleitung und einen Substituatport sowie eine Blut führende Leitung als Teil eines extrakorporalen Blutkreislauf (200) aufweist, wobei der Substituatport vorgesehen ist, um von der Hydraulik (1) der Blutbehandlungsvorrichtung (100) erzeugtes Substituat zu empfangen, wobei die medizinische Funktionsvorrichtung keine Vorrichtung aufweist, welche angeordnet und/oder vorgesehen ist für eine Dosierung des von der Substituatleitung in eine Blut führende Leitung der Blutkassette übertretenden Substituats; und mit
- einer Steuer- oder Regelvorrichtung (300), konfiguriert oder programmiert zum Durchführen eines Verfahrens zum Dosieren, Erzeugen oder Bereitstellen eines mittels einer Blutbehandlungsvorrichtung (100) erzeugten Substituats, wobei das Dosieren mittels der Hydraulik (1) der Blutbehandlungsvorrichtung (100) erfolgt;
wobei das Verfahren die Schritte umfasst:
- Fördern eines ersten Fluids durch die erste Filterstufe (F04) hindurch in die Dialysierflüssigkeitszulaufleitung (3) unter Erzeugen einer in den Dialysator (5) einbringbaren Dialysierflüssigkeit;
- Leiten eines Anteils der Dialysierflüssigkeit in eine Substituatleitung (9), welche sich an die zweite Filterstufe (F05) anschließt oder hiermit in Fluidkommunikation steht, unter Erzeugen eines in einen extrakorporalen Blutkreislauf (200) einbringbaren Substituats bei Durchtritt des Dialysats durch die zweite Filterstufe (F05); und
- Regeln oder Steuern der Größe des Anteils der Dialysierflüssigkeit, welcher die zweite Filterstufe (F05) durchtritt, oder des Anteils, welcher nach Filterung an der zweiten Filterstufe (F05) vorgesehen ist über die Substituatleitung (9) in die Blutkassette eingeführt zu werden, wobei das Regeln oder Steuern durch Einwirken auf wenigstens eine Fördervorrichtung (11, 12, 31), auf wenigstens eine Flussbegrenzungseinrichtung (Vdia, Vsub, 21) oder auf ein Flussteilerventil (37), welche jeweils in der Dialysierflüssigkeitszulaufleitung (3) und/oder der Substituatleitung (9) und/oder in der die Dialysierflüssigkeitszulaufleitung (3) mit der Substituatleitung (9) verbindenden Abzweigleitung (35) vorliegen oder auf diese wirken, erfolgt.

2. Blutbehandlungsvorrichtung (100) nach Anspruch 1, wobei die Abzweigleitung (35) in die zweite Filterstufe (F05) mündet.

3. Blutbehandlungsvorrichtung (100) nach einem der vorangegangenen Ansprüchen, wobei das auf diese Weise erzeugte Substituat ohne weitere Maßnahmen, ohne Verändern seiner Zusammensetzung oder ohne Verändern seiner Förderung, welche einem Dosieren des in den extrakorporalen Blutkreislauf (200) eingebrachten oder einzubringenden Substituats dienten, in den extrakorporalen Blutkreislauf (200) eingebracht wird.

4. Blutbehandlungsvorrichtung (100) nach einem der vorangegangenen Ansprüchen, wobei das Verfahren den weiteren Schritt umfasst:
- Regeln oder Steuern der Größe des Anteils, welcher die zweite Filterstufe (F05) durchtritt oder in dieser filtriert wird, basierend auf voreingestellten Angaben zum gewünschten Substituatfluss oder Substituatvolumen oder basierend auf während der Behandlung ermittelten Daten, aus welchen in einem Schritt des Verfahrens Angaben über den erforderlichen Substituatfluss errechnet werden.

5. Blutbehandlungsvorrichtung (100) nach einem der vorangegangenen Ansprüchen, wobei die zweite Filterstufe (F05) in die Dialysierflüssigkeitszulaufleitung (3) eingefügt ist;
wobei in der Dialysierflüssigkeitszulaufleitung (3) stromabwärts der zweiten Filterstufe (F05) ein Proportionalventil (Vdia) angeordnet ist; und
wobei in der Substituatleitung (9) stromabwärts der zweiten Filterstufe (F05) ein Proportionalventil (Vsub) oder eine Drossel (21) angeordnet ist.

6. Blutbehandlungsvorrichtung (100) nach einem der vorangegangenen Ansprüchen, wobei die zweite Filterstufe (F05) in die Dialysierflüssigkeitszulaufleitung (3) eingefügt ist; und
wobei in der Substituatleitung (9) stromabwärts der zweiten Filterstufe (F05) eine Druckpumpe (12) angeordnet ist.

7. Blutbehandlungsvorrichtung (100) nach Anspruch 6, wobei in der Substituatleitung (9) stromabwärts der Druckpumpe (12) ein Temperatursensor (23) angeordnet ist, ein Partikelfilter (25) angeordnet ist, oder eine Bypassleitung (27) von der Substituatleitung (9) abgeht.

8. Blutbehandlungsvorrichtung (100) nach einem der vorangegangenen Ansprüchen,
wobei die zweite Filterstufe (F05) in die Dialysierflüssigkeitszulaufleitung (3) eingefügt ist; und
wobei in der Substituatleitung (9) stromabwärts der zweiten Filterstufe (F05) eine Volumenpumpe (31) angeordnet ist.

9. Blutbehandlungsvorrichtung (100) nach Anspruch 8, wobei in der Substituatleitung (9) stromaufwärts der Volumenpumpe (31) ein Substituatdrucksensor (29), stromabwärts der Volumenpumpe (31) ein Partikelfilter (25) und/oder ein Drucksensor (15), angeordnet ist.

10. Blutbehandlungsvorrichtung (100) nach einem der vorangegangenen Ansprüchen, wobei in der Dialysierflüssigkeitszulaufleitung (3) oder in der Substituatleitung (9) wenigstens ein Flusssensor (19a, 19b) angeordnet ist.

11. Blutbehandlungsvorrichtung (100) nach einem der vorangegangenen Ansprüchen, wobei von der Dialysierflüssigkeitszulaufleitung (3) an einem Abzweigungspunkt (35a) die Abzweigleitung (35) abgeht, welche stromabwärts des Abzweigungspunkts (35a) in die zweite Filterstufe (F05) mündet, wobei aus der zweiten Filterstufe (F05) die Substituatleitung (9) hervorgeht; und
wobei im Abzweigungspunkt (35a) und damit stromaufwärts der zweiten Filterstufe (F05) ein Flussteilerventil (37) angeordnet ist.

12. Blutbehandlungsvorrichtung (100) nach einem der vorangegangenen Ansprüchen, wobei von der Dialysierflüssigkeitszulaufleitung (3) an einem Abzweigungspunkt (35a) eine Abzweigleitung (35) abgeht, welche stromabwärts des Abzweigungspunkts (35a) in die zweite Filterstufe (F05) mündet, wobei aus der zweiten Filterstufe (F05) die Substituatleitung (9) hervorgeht;
wobei in der Dialysierflüssigkeitszulaufleitung (3) stromabwärts des Abzweigungspunkts (35a) ein Proportionalventil (Vdia) angeordnet ist, oder
wobei in der Abzweigleitung (35) stromaufwärts der zweiten Filterstufe (F05) ein Proportionalventil (Vsub) oder eine Drossel (21) angeordnet ist.

13. Blutbehandlungsvorrichtung (100) nach einem der vorangegangenen Ansprüchen, wobei von der Dialysierflüssigkeitszulaufleitung (3) an einem Abzweigungspunkt (35a) eine Abzweigleitung (35) abgeht, welche stromabwärts des Abzweigungspunkts (35a) in die zweite Filterstufe (F05) mündet, wobei aus der zweiten Filterstufe (F05) die Substituatleitung (9) hervorgeht; und
wobei in der Abzweigleitung (35) stromabwärts des Abzweigungspunkts (35a) eine Vordruckpumpe (11) angeordnet ist.

14. Blutbehandlungsvorrichtung (100) nach Anspruch 13, wobei stromabwärts der in der Abzweigleitung (35) gelegenen Vordruckpumpe (11) ein Temperatursensor (23) angeordnet ist.

15. Blutbehandlungsvorrichtung (100) nach einem der vorangegangenen Ansprüchen, wobei von der Dialysierflüssigkeitszulaufleitung (3) an einem Abzweigungspunkt (35a) eine Abzweigleitung (35) abgeht, welche stromabwärts des Abzweigungspunkts (35a) in die zweite Filterstufe (F05) mündet, wobei aus der zweiten Filterstufe (F05) die Substituatleitung (9) hervorgeht; und
wobei in der Abzweigleitung (35) stromabwärts des Abzweigungspunkts (35a) eine Volumenpumpe (31) angeordnet ist, und wobei stromabwärts der Volumenpumpe (31) aber stromaufwärts der zweiten Filterstufe (F05) ein Abzweigleitungsdrucksensor (16) vorgesehen ist.

16. Blutbehandlungsvorrichtung (100) nach einem der vorangegangenen Ansprüchen, wobei von der Dialysierflüssigkeitszulaufleitung (3) an einem Abzweigungspunkt (35a) eine Abzweigleitung (35) abgeht, welche stromabwärts des Abzweigungspunkts (35a) in die zweite Filterstufe (F05) mündet, wobei aus der zweiten Filterstufe (F05) die Substituatleitung (9) hervorgeht; und
wobei stromaufwärts des Abzweigungspunkts (35a) eine Vordruckpumpe (11) angeordnet ist, und/oder wobei von der zweiten Filterstufe (F05) eine Flushleitung (28) abgeht.

17. Blutbehandlungsvorrichtung (100) nach einem der vorangegangenen Ansprüchen, wobei in der Substituatleitung (9) oder der Dialysierflüssigkeitszulaufleitung (3) stromabwärts des Abzweigungspunkts (35a) oder stromabwärts der zweiten Filterstufe (F05) oder in der Abzweigleitung (35) wenigstens ein Flusssensor (19a, 19b, 19c) angeordnet ist.

## Claims

1. A blood treatment apparatus (100) with
- a hydraulic system (1) comprising at least one first filtration stage (F04) and one second filtration stage (F05), comprising at least one conveying apparatus (11, 12, 31), at least one flow limitation device (Vdia, Vsub, 21) or a flow divider valve (37), as well as comprising at least one dialysis liquid supply line (3) and at least one substituate line (9), as well as optionally at least one branch line (35) which connects the dialysis liquid supply line (3) and the substituate line (9) ;
- a medical functional apparatus embodied as blood cassette comprising a substituate line and a substituate port as well as a blood conducting-line which is part of an extracorporeal blood circuit (200), wherein the substituate port is provided to receive substituate produced by the hydraulic system (1) of the blood treatment apparatus (100), wherein the medical functional apparatus does not comprise any apparatus which is arranged and/or provided for dosing the substituate passing over from the substituate line into a blood-conducting line of the blood cassette; and
- a control or regulating apparatus (300), configured or programmed to execute a method for dosing, creating or providing a substituate which was produced by a blood treatment apparatus (100), wherein dosing takes place by the hydraulic system (1) of the blood treatment apparatus (100);
wherein the method comprises the steps:
- conveying a first fluid through the first filtration stage (F04) into the dialysis liquid supply line (3), producing or generating a dialysis liquid which is introducible into the dialyzer (5);
- conducting a portion of the dialysis liquid into a substituate line (9) which attaches to the second filtration stage (F05) or is in fluid communication with it, producing or generating a substituate which is introducible into an extracorporeal blood circuit (200) when the dialysate passes through the second filtration stage (F05); and
- regulating or controlling the size of the portion of the dialysis liquid which passes through the second filtration stage (F05), or of the portion which after filtration at the second filtration stage (F05) is provided to be introduced into the blood cassette via the substituate line (9), wherein regulating or controlling takes place by affecting at least one conveying apparatus (11, 12, 31), at least one flow limitation device (Vdia, Vsub, 21) or a flow divider valve (37), which are each present in the dialysis liquid supply line (3) and/or in the substituate line (9) and/or in the branch line (35) which connects the dialysis liquid supply line (3) and the substituate line (9) or affect these.

2. The blood treatment apparatus (100) according to claim 1, wherein the branch line (35) ends in the second filtration stage (F05).

3. The blood treatment apparatus (100) according to anyone of the preceding claims, wherein the substituate which is produced this way is introduced into the extracorporeal blood circuit (200) without any further measures, without changing its composition or without changing its conveyance which served dosing of the substituate which was introduced or is to be introduced into the extracorporeal blood circuit (200).

4. The blood treatment apparatus (100) according to anyone of the preceding claims, wherein the method further encompasses the step:
- regulating or controlling the size of the portion which passes through the second filtration stage (F05) or which is filtrated in it, based on preset information regarding the desired substituate flow or substituate volume, or based on data which was determined during the treatment, from which in a step of the method information about the required substituate flow is calculated.

5. The blood treatment apparatus (100) according to anyone of the preceding claims, wherein the second filtration stage (F05) is integrated in the dialysis liquid supply line (3) ;
wherein in the dialysis liquid supply line (3) downstream of the second filtration stage (F05) a proportional valve (Vdia) is arranged; and
wherein a proportional valve (Vsub) or a throttle (21) is arranged in the substituate line (9) downstream of the second filtration stage (F05).

6. The blood treatment apparatus (100) according to anyone of the preceding claims, wherein the second filtration stage (F05) is integrated in the dialysis liquid supply line (3); and
wherein a pressure pump (12) is arranged in the substituate line (9) downstream of the second filtration stage (F05).

7. The blood treatment apparatus (100) according to claim 6, wherein a temperature sensor (23), a particle filter (25) is arranged in the substituate line (9) downstream of the pressure pump (12), or a bypass line (27) branches off the substituate line (9).

8. The blood treatment apparatus (100) according to anyone of the preceding claims, wherein the second filtration stage (F05) is integrated in the dialysis liquid supply line (3); and
wherein a volume pump (31) is arranged in the substituate line (9) downstream of the second filtration stage (F05).

9. The blood treatment apparatus (100) according to claim 8, wherein a substituate pressure sensor (29) is arranged in the substituate line (9) upstream of the volume pump (31), a particle filter (25) and/or a pressure sensor (15) is arranged downstream of the volume pump (31).

10. The blood treatment apparatus (100) according to anyone of the preceding claims, wherein at least one flow sensor (19a, 19b) is arranged in the dialysis liquid supply line (3) or in the substituate line (9).

11. The blood treatment apparatus (100) according to anyone of the preceding claims, wherein the branch line (35) branches off from the dialysis liquid supply line (3) at a branch point (35a), which leads into the second filtration stage (F05) downstream from the branch point (35a), wherein the substituate line (9) emerges from the second filtration stage (F05);
wherein a flow divider valve (37) is arranged in the branch point (35a) and thus upstream of the second filtration stage (F05).

12. The blood treatment apparatus (100) according to anyone of the preceding claims, wherein a branch line (35) branches off from the dialysis liquid supply line (3) at a branch point (35a), which leads downstream of the branch point (35a) into the second filtration stage (F05), wherein the substituate line (9) emerges from the second filtration stage (F05);
wherein a proportional valve (Vdia) is arranged in the dialysis liquid supply line (3) downstream of the branch point (35a), or wherein a proportional valve (Vsub) or a throttle (21) is arranged in the branch line (35) upstream of the second filtration stage (F05).

13. The blood treatment apparatus (100) according to anyone of the preceding claims, wherein a branch line (35) branches off from the dialysis liquid supply line (3) at a branch point (35a), which leads into the second filtration stage (F05) downstream of the branch point (35a), wherein the substituate line (9) emerges from the second filtration stage (F05); and
wherein a pre-pressure pump (11) is arranged in the branch line (35) downstream of the branch point (35a).

14. The blood treatment apparatus (100) according to claim 13, wherein a temperature sensor (23) is arranged downstream of the pre-pressure pump (11) which is located in the branch line (35).

15. The blood treatment apparatus (100) according to anyone of the preceding claims, wherein a branch line (35) branches off from the dialysis liquid supply line (3) at a branch point (35a), which leads into the second filtration stage (F05) downstream of the branch point (35a), wherein the substituate line (9) emerges from the second filtration stage (F05); and
wherein a volume pump (31) is arranged in the branch line (35) downstream of the branch point (35a), and wherein a branch line pressure sensor (16) is provided downstream of the volume pump (31) but upstream of the second filtration stage (F05).

16. The blood treatment apparatus (100) according to anyone of the preceding claims, wherein a branch line (35) branches off from the dialysis liquid supply line (3) at a branch point (35a), wherein the branch line (35a) leads into the second filtration stage (F05) downstream of the branch point (35a), wherein the substituate line (9) emerges from the second filtration stage (F05); and
wherein a pre-pressure pump (11) is arranged upstream of the branch point (35a), and/or wherein a flush line (28) branches off the second filtration stage (F05).

17. The blood treatment apparatus (100) according to anyone of the preceding claims, wherein at least one flow sensor (19a, 19b, 19c) is arranged in the substituate line (9) or in the dialysis liquid supply line (3) downstream of the branch point (35a) or downstream of the second filtration stage (F05) or in the branch line (35).

## Revendications

1. Un appareil de traitement du sang (100) comprenant :
- un système hydraulique (1) comprenant au moins un premier étage de filtrage (F04) et un second étage de filtrage (F05), au moins un appareil de transport (11, 12, 31), au moins un dispositif de limitation de débit (Vdia, Vsub, 21) ou une vanne diviseur de flux (37), ainsi qu'au moins un conduit d'entrée du liquide de dialyse (3) et au moins un conduit pour le substitut (9), ainsi que, de façon optionnelle, au moins un conduit de dérivation (35) reliant le conduit d'entrée du liquide de dialyse (3) au conduit de substitut (9) ;
- un appareil médical fonctionnel conçu comme une cassette à sang comprenant un conduit de substitut et un terminal de connexion du substitut ainsi qu'un conduit de circulation du sang faisant partie d'un circuit extracorporel de circulation du sang (200), où le terminal de connexion du substitut est prévu pour recevoir le substitut produit par le système hydraulique (1) de l'appareil de traitement du sang (100), où l'appareil médical fonctionnel ne comprend aucun appareil conçu et/ou prévu pour doser le substitut passant du conduit de substitut au conduit de circulation du sang; et
- un appareil de commande ou de régulation (300), configuré ou programmé pour exécuter un procédé prévu pour doser, produire ou mettre à disposition un substitut produit au moyen d'un appareil de traitement du sang (100), où le dosage a lieu au moyen du système hydraulique (1) de l'appareil de traitement du sang (100) ;
où le procédé comprend les étapes suivantes :
- acheminer un premier fluide au travers du premier étage de filtrage (F04) vers le conduit d'entrée du liquide de dialyse (3) produisant ainsi un liquide de dialyse pouvant être introduit dans le dialyseur (5);
- diriger une partie du liquide de dialyse vers un conduit de substitut (9) connecté au second étage de filtrage (F05), ou étant en communication de fluide avec celui-ci, produisant ainsi un substitut pouvant être introduit dans un circuit extracorporel de circulation du sang (200) lors du passage du dialysat au travers du second étage de filtrage (F05); et
- réguler ou commander la part de liquide de dialyse passant le second étage de filtrage (F05), ou la part destinée à être introduite dans la cassette à sang via le conduit de substitut (9) après filtration au niveau du second étage de filtrage (F05), où la régulation ou la commande a lieu par action sur au moins un appareil de transport (11, 12, 13), au moins un dispositif de limitation de débit (Vdia, Vsub, 21) ou une vanne diviseur de flux (37), chacun étant présents dans le conduit d'entrée du liquide de dialyse (3) et/ou dans le conduit de substitut (9) et/ou dans le conduit de dérivation (35) reliant le conduit d'entrée du liquide de dialyse (3) au conduit de substitut (9) ou agissant sur ceux-ci.

2. L'appareil de traitement du sang (100) selon la première revendication, où le conduit de dérivation (35) débouche dans le second étage de filtrage (F05).

3. L'appareil de traitement du sang (100) selon l'une quelconque des revendications précédentes, où le substitut produit de cette façon est introduit dans le circuit extracorporel de circulation du sang (200) sans mesures supplémentaires, sans changement de sa composition ou de son acheminement, mesures qui servaient à doser le substitut introduit ou devant être introduit dans le circuit extracorporel de circulation du sang (200).

4. L'appareil de traitement du sang (100) selon l'une quelconque des revendications précédentes, où le procédé comprend l'étape supplémentaire suivante :
- réguler ou commander la part qui passe le second étage de filtrage (F05) ou qui est filtrée dans celui-ci, sur la base d'informations préréglées concernant le flux désiré du substitut ou le volume du substitut ou sur la base de données recueillies lors du traitement, à partir desquelles des informations concernant le flux de substitut nécessaire sont calculées dans une étape du procédé.

5. L'appareil de traitement du sang (100) selon l'une quelconque des revendications précédentes, où le second étage de filtrage (F05) est inséré dans le conduit d'entrée du liquide de dialyse (3);
où une vanne proportionnelle (Vdia) est agencée en aval du second étage de filtrage (F05) dans le conduit d'entrée du liquide de dialyse (3); et
où une vanne proportionnelle (Vsub) ou une vanne d'étranglement (21) est agencée en aval du second étage de filtrage (F05) dans le conduit de substitut (9).

6. L'appareil de traitement du sang (100) selon l'une quelconque des revendications précédentes, où le second étage de filtrage (F05) est inséré dans le conduit d'entrée du liquide de dialyse (3); et
où une pompe à pression (12) est agencée en aval du second étage de filtrage (F05) dans le conduit de substitut (9).

7. L'appareil de traitement du sang (100) selon la revendication 6, où une sonde de température (23), un filtre à particules (25), est agencé en aval de la pompe à pression (12) dans le conduit de substitut (9), ou où un conduit de dérivation (27) sort du conduit de substitut (9).

8. L'appareil de traitement du sang (100) selon l'une quelconque de revendications précédentes,
où le second étage de filtrage (F05) est inséré dans le conduit d'entrée du liquide de dialyse (3) ; et où une pompe volumétrique (31) est agencée en aval du second étage de filtrage (F05) dans le conduit de substitut (9).

9. L'appareil de traitement du sang (100) selon la revendication 8, où, dans le conduit de substitut (9), un capteur de pression de substitut (29) est agencé en amont de la pompe volumétrique (31), un filtre à particules (25) et/ou un capteur de pression (15) est/sont agencé (s) en aval de la pompe volumétrique (31).

10. L'appareil de traitement du sang (100) selon l'une quelconque des revendications précédentes, où au moins un capteur de débit (19a, 19b) est agencé dans le conduit d'entrée du liquide de dialyse (3) ou dans le conduit de substitut (9).

11. L'appareil de traitement du sang (100) selon l'une quelconque des revendications précédentes, où le conduit de dérivation (35) sort du conduit d'entrée du liquide de dialyse (3) à un point d'embranchement (35a) et débouche dans le second étage de filtrage (F05) en aval du point d'embranchement (35a), où le conduit de substitut (9) ressort du second étage de filtrage (F05); et
où une vanne diviseur de flux (37) est agencée au point d'embranchement (35a) et donc en amont du second étage de filtrage (F05).

12. L'appareil de traitement du sang (100) selon l'une quelconque des revendications précédentes, où un conduit de dérivation (35) sort du conduit d'entrée du liquide de dialyse (3) à un point d'embranchement (35a) et débouche dans le second étage de filtrage (F05) en aval du point d'embranchement (35a), où le conduit de substitut (9) ressort du second étage de filtrage (F05);
où une vanne proportionnelle (Vdia) est agencée dans le conduit d'entrée du liquide de dialyse (3) en aval du point d'embranchement (35a), ou où une vanne proportionnelle (Vsub) ou une vanne d'étranglement (21) est agencée dans le conduit de dérivation (35) en amont du second étage de filtrage (F05).

13. L'appareil de traitement du sang (100) selon l'une quelconque des revendications précédentes, où un conduit de dérivation (35) sort du conduit d'entrée du liquide de dialyse (3) à un point d'embranchement (35a) et débouche dans le second étage de filtrage (F05) en aval du point d'embranchement (35a), où le conduit de substitut (9) ressort du second étage de filtrage (F05); et
où une pompe d'amorçage (11) est agencée dans le conduit de dérivation (35) en aval du point d'embranchement (35a).

14. L'appareil de traitement du sang (100) selon la revendication 13, où une sonde de température (23) est agencée en aval de de la pompe d'amorçage (11) située dans le conduit de dérivation (35).

15. L'appareil de traitement du sang (100) selon l'une quelconque des revendications précédentes, où un conduit de dérivation (35) sort du conduit d'entrée du liquide de dialyse (3) à un point d'embranchement (35a) et débouche dans le second étage de filtrage (F05) en aval du point d'embranchement (35a), où le conduit de substitut (9) ressort du second étage de filtrage (F05); et
où une pompe volumétrique (31) est agencée dans le conduit de dérivation (35) en aval du point d'embranchement (35a), et où un capteur de pression du conduit de dérivation (16) est prévu en aval de la pompe volumétrique (31) mais en amont du second étage de filtrage (F05).

16. L'appareil de traitement du sang (100) selon l'une quelconque des revendications précédentes, où un conduit de dérivation (35) sort du conduit d'entrée du liquide de dialyse (3) à un point d'embranchement (35a) et débouche dans le second étage de filtrage (F05) en aval du point d'embranchement (35a), où le conduit de substitut (9) ressort du second étage de filtrage (F05); et
où une pompe d'amorçage (11) est agencée en amont du point d'embranchement (35a), et/ou où un conduit de rinçage (28) sort du second étage de filtrage (F05).

17. L'appareil de traitement du sang (100) selon l'une quelconque des revendications précédentes, où au moins un capteur de débit (19a, 19b, 19c) est agencé dans le conduit de substitut (9) ou dans le conduit d'entrée du liquide de dialyse (3) en aval du point d'embranchement (35a), en aval du second étage de filtrage (F05) ou dans le conduit de dérivation (35).
